(19) Europäisches Patentamt / European Patent Office / Office européen des brevets

(11) **EP 3 501 399 B1**

(12) **EUROPEAN PATENT SPECIFICATION**

(45) Date of publication and mention of the grant of the patent:
**14.10.2020  Bulletin 2020/42**

(51) Int Cl.:
*A61B 6/03* *(2006.01)*      *A61B 6/00* *(2006.01)*
*A61B 5/00* *(2006.01)*      *G06T 7/60* *(2017.01)*

(21) Application number: **17382876.5**

(22) Date of filing: **21.12.2017**

(54) **METHOD OF QUANTIFICATION OF VISCERAL FAT MASS**

VERFAHREN ZUR QUANTIFIZIERUNG DER VISZERALEN FETTMASSE

PROCÉDÉ DE QUANTIFICATION D'UNE MASSE DE GRAISSE VISCÉRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.06.2019  Bulletin 2019/26**

(73) Proprietor: **Cetir Centre Mèdic S.L.**
**08029 Barcelona (ES)**

(72) Inventors:
• **DEL RÍO BARQUERO, Luis Miguel**
  **08029 Barcelona (ES)**
• **CHARY, Gaëtan**
  **08014 Barcelona (ES)**
• **DI GREGORIO, Silvana**
  **08026 Barcelona (ES)**

• **ROMERO MARTÍN, Juan Antonio**
  **46100 Busjassot (Valencia) (ES)**

(74) Representative: **Pons Ariño, Angel**
**Pons Patentes y Marcas Internacional, S.L.**
**Glorieta Rubén Dario 4**
**28010 Madrid (ES)**

(56) References cited:
**WO-A2-2011/098895      US-A1- 2011 158 386**

• **SANJIV KAUL ET AL: "Dual-Energy X-Ray Absorptiometry for Quantification of Visceral Fat", OBESITY RESEARCH, vol. 20, no. 6, 26 January 2012 (2012-01-26), pages 1313-1318, XP055477226, US ISSN: 1930-7381, DOI: 10.1038/oby.2011.393**

**Description**

**OBJECT OF THE INVENTION**

The object of the invention pertains to the technical field of diagnostic imaging.

More specifically, this document describes a methodology that enables the amount of visceral fat mass in a living being to be determined in a non-invasive manner.

**BACKGROUND OF THE INVENTION**

Matters related to the cardio-metabolic risk between different phenotypes, along with the increase of risks associated with visceral fat, clearly indicate the need to measure visceral adipose tissue as differentiating element of significant health risk in the population who are overweight or obese.

The metabolic causes and consequences of a specific distribution of fat deposits have particular clinical relevance since they may entail serious health risks. Therefore, the aim for a suitable, preventative action is to identify the behavior and distribution of the abdominal adipose tissue as well as the fatty infiltration of muscles and organs when diagnosing the risks it entails at a subject-specific level.

Until now, visceral fat was examined by means of three-dimensional medical imaging techniques such as Computed Tomography or Magnetic Resonance Imaging, both of which are expensive techniques, with high doses of radiation in the first case and with a very long duration and causing discomfort to the patient in the second case. However, recent studies support the use of Dual-Energy X-Ray Absorptiometry (DXA), commonly known as Densitometry, attaining high correlation indexes with said techniques when quantifying visceral fat, despite the fact that said studies are based on the conventional analysis of a single scan and a statistical estimation of the amount of fat mass, which is useful for group studies but not at a patient-specific level.

Said methods for estimating the amount of fat mass include those specifically intended to determine levels of visceral fat (VAT or visceral adipose tissue), which can be used as a basis for diagnosing or treating different diseases related to levels of intra-abdominal adipose tissue, which is the fat located at a deeper level than the subcutaneous level where subcutaneous adipose tissue (SAT) is located; the visceral fat (VAT) being the fat that surrounds the internal organs of the abdominal cavity, such that the excess of visceral fat (VAT) deposits is associated with health problems, such as cardiovascular diseases, hypertension and metabolic diseases such as type 2 diabetes.

Radiodiagnostic techniques that allow volumetric reconstruction are normally used to determine abdominal fat levels, however, they imply high doses of radiation during an excessive period of time, which may lead to other health problems and/or entail a high economic cost.

Furthermore, in diagnostic imaging there is a common and known method which is dual-energy or double-energy X-ray absorptiometry, called DXA/DEXA; this methodology is widely accepted in branches of science related to the diagnosis and clinical management of metabolic bone diseases that require the assessment of bone density. DXA is commonly used to diagnose osteoporosis related to a gradual loss of calcium, generating structural changes, and thus causing bones to lose thickness, become more fragile and be more likely to break.

However, DXA is not limited to only measuring bone density, and other applications are known from the document by Albanese et al., Clinical applications of body composition measurements using DXA; Journal of Clinical Densitometry 2003. Among others, the measurement of body composition is described and, in particular, the quantification of fat mass by means of the DXA full-body scan.

In this regard, the document US7725153B2 describes a methodology that uses X-rays to determine the internal characteristics of patients or objects, and in a specific implementation described in this document, the use of dual-energy X-ray absorptiometry to determine visceral fat content and to use the results obtained is described. This document describes a problem already known and mentioned above; Computed Tomography (CT) and Magnetic Resonance Imaging (MRI) are currently used to measure or estimate VAT, distinguishing it from SAT in abdominal cross sections or scans. This measurement is usually made at the level of the navel, wherein the SAT and VAT volumes are identified by means of an image thresholding algorithm. However, the relatively high cost of both exams, the high dose of radiation from the CT and the need for segmentation and subsequent calculation tasks of the generated scans require a long processing time and may discourage the use of these techniques as a tool for detecting VAT levels.

The document US8483458B2 discloses a system for measuring visceral fat mass and a method for acquiring dual-energy (DXA) two-dimensional (2D) scan information from a dual-energy X-ray scan of a body and generating an image of the body using the 2D scan information. The method described includes identifying a region of interest using the dual-energy X-ray image and determining a subcutaneous fat mass for each of a plurality of sections of the region of interest. The method also includes determining the visceral fat mass for the region of interest based on the determined subcutaneous fat mass for each of the plurality of sections.

Likewise, the document A Mohammad et al., Validity of visceral adiposity estimates from DXA against MRI in Kuwaiti men and women; Nutrition & Diabetes 7, e238 9/1/2017, describes a study on the possibility of estimating VAT by using DXA-based techniques. This document describes, among other aspects, the validity of said techniques for estimating visceral fat (VAT), while making it clear that accuracy may possibly decrease with the increasing level of visceral adiposity. It also describes the use of a full-body scan to carry out the DXA scan, although it also describes how it calculates the value of visceral fat (VAT), which is obtained from the value of total abdominal fat (TAF) from which the measurement of the subcutaneous adipose tissue (SAT) is subtracted; in other words, the total fat and subcutaneous fat (SAT) fat are quantified and the value of visceral fat (VAT) is obtained by subtraction. The document Sanjiv Kaul et al., Dual-Energy X-Ray Absorptiometry for Quantification of Visceral Fat, Obesity (Silver Spring) 2012 Jun;20(6):1313-8 discloses a method for quantifying visceral fat using dual-energy X-ray absorptiometry based on geometric constants and assumptions derived from CT dat but without determination of the abdominal depth and of the abdominal cavity depth.

In the methodologies known in the prior art, simple geometric models that statistically extrapolate the volumetric amount from 2D images and, in some cases, 3D models based on a single CT or MRI scan are used. The most recent studies validate the idea that three-dimensional modeling of the body and of the distribution of tissues provide more information than conventional densitometric images and allow risks associated with pathologies, such as metabolic syndrome, to be predicted. Therefore, basic geometric models, either square/rectangular or ellipsoidal, must be used in the state of the art.

Finally, it is worth noting that the object of the invention has a greater scope in the application thereof. In addition to enabling the analysis of visceral fat in the abdominal cavity, the methodology can be applied to other regions of the human or animal body in which fatty infiltration in tissues may be a risk factor that triggers other types of pathologies and health problems, as well as in the industry to analyze pieces of a biological source.

One example is fatty infiltration of muscles that occurs at a macroscopic level, taking place between the fiber bundles and between muscle groups and/or at a microscopic level, inside the myocytes.

This type of infiltration, when it exceeds the normal limits, leads to sarcopenic obesity, a pathological condition in which the loss of muscle mass in volume and functionality is masked by the increase of body volume/weight due to the increase of the fat compartment, or when the ratio of fat mass exceeds the ratio of non-fat tissue, regardless of the destruction of muscle fibers.

In light of the above, it is deduced that the quantification of the muscle mass and of the fat mass, both in absolute terms and in terms of the ratio therebetween, is of vital clinical significance in order to categorize the subjects at risk of suffering from the harmful effects thereof.

Likewise, obtaining an indirect marker of muscular function, such as fatty infiltration of muscles, complements the assessment of the muscular state of individuals and makes it easier to diagnose sarcopenia.

Therefore, having a method that enables both components and, in particular, the fat mass to be identified as a tissue producing inflammation factors is of vital importance, since it will provide information that is difficult to routinely attain in clinical practice, thus significantly contributing to the identification, monitoring and assessment of population groups at risk of fragility.

## DESCRIPTION OF THE INVENTION

The object of the present invention is a method for estimating fat in the abdominal cavity (known as visceral fat) which may be useful in data collection applications used to make decisions related to the prediction of cardiovascular risk and metabolic syndrome. The method object of the invention is based on volumetric reconstruction techniques by means of medical imaging of dual-energy X-ray absorptiometry (DXA). The method object of the invention provides an optimal solution to the aforementioned problem, since it enables obtaining the values of the amount of fat mass very similar to those obtainable when using methodologies that are slower or more harmful to human beings due to the large amount of radiation that they can require.

Therefore, the method object of the invention uses a subject-specific abdominal recognition module with three-dimensional geometry that calculates the distribution of abdominal fat deposits thanks to the generation of 3D models which are obtained from a 2D image extracted from the densitometer and a 3D image extracted from the Computed Tomography (CT), with the aim of reconstructing the abdominal cavity to subsequently assess the distribution of visceral (VAT) and subcutaneous fat (SAT). From said data, the amount of fat mass is calculated, identifying the location thereof in the abdominal cavity. To carry out the method for estimating visceral fat (VAT) object of the invention, variable geometric models are used that are not only ellipsoidal, but also hyper-ellipsoidal geometric models or others that better adapt to the area of study in which the amount of visceral fat mass is to be estimated.

The estimation is carried out in such a way that the calculation to quantify the visceral fat (VAT) mass is carried out by calculating the subcutaneous fat (SAT) mass, based on the estimation of the subcutaneous thickness and a percentage of fat contained in the subcutaneous tissue, and subtracting it from the total fat mass obtained from the DXA image. In the case of obtaining a negative value of visceral fat (VAT) mass, it is corrected by assigning it a null amount to avoid overestimating the visceral fat (VAT) that may lead to false positives; thus avoiding the overestimation of the visceral fat (VAT) mass over the overestimation of the subcutaneous fat (SAT) mass which is the most suitable from the point of view of the application of the object of the invention.

Volumes are calculated for a set of coordinate values (x,y) corresponding to a selected region of interest (ROI). The volume corresponding to each coordinate (x,y) is determined based on values of the heights $H(x,y)$ and $h(x,y)$, which correspond to the height of each coordinate (x,y) with respect to a reference, such as a surface like the gurney on which the subject rests during imaging. To obtain the value of these functions, a database of geometries of the abdomen and of the abdominal cavity of known patients obtained from CT or MRI scans, and in which the value of $H(x,y)$ and $h(x,y)$ has been previously measured, can be used. The geometries that will be used to calculate the volume of the subject under study are those with a greater degree of similarity to the geometry of this subject, according to the abdomen and abdominal cavity diameter at different heights of the ROI and according to the abdomen and abdominal cavity depth.

Once the values of the functions $H(x,y)$ and $h(x,y)$ are obtained, the tissue thickness corresponding to the subcutaneous fat (SAT) mass $e(X,Y)$ is estimated. To estimate the subcutaneous fat (SAT) mass, the thickness thereof is multiplied by the dimensions given by the resolution $Rx*Ry$ of the DXA images, the fat density $\rho_{Fat}$ and the ratio of fat in the subdermal tissue $\%G_{Subd}$. This final data is obtained by measuring the ratio of subcutaneous fat (SAT) mass in the area exclusively composed of subcutaneous tissue and located on the sides of the abdomen in the density processed fat (DPF) image.

Thus, the thickness of the tissue corresponding to subcutaneous fat (SAT) underlying the pixel of coordinates (X,Y), $e(x,y)$ is calculated by means of:

$$e(x,y) = H(x,y) - h(x,y)$$

The amount of subcutaneous fat (SAT) mass in the pixel (x,y) $M_{SAT(x,y)}$ is calculated by means of:

$$M_{SAT(x,y)} = [\%G_{SAT}].Rx.Ry.\rho_{Fat}. (H(x,y) - h(x,y))$$

The amount of visceral fat (VAT) mass in the pixel (x,y), underlying the abdominal cavity, $M_{VAT(x,y)}$ is calculated by means of:

$$M_{VAT(x,y)} = M_{(x,y)} - [\%G_{SAT}].Rx.Ry.\rho_{Fat}. (H(x,y) - h(x,y))$$

To obtain the total values in the ROI:
The amount of subcutaneous fat (SAT) mass throughout the entire ROI is obtained in the following way:

$$M_{SAT} = [\%G_{SAT}].Rx.Ry.\rho_{Fat}.\Sigma_{ROI} (H-h)$$

The total amount of visceral fat (VAT) mass throughout the entire ROI is obtained in the following way:

$$M_{VAT} = \Sigma_{Cavity}M_{(x,y)} - [\%G_{SAT}].Rx.Ry.\rho_{Fat}.\Sigma_{Cavity}(H-h)$$

Alternatively, the total amount of visceral fat (VAT) mass can be obtained by subtraction from the total amount of fat:

$$M_{Total} = \Sigma_{ROI} M_{(x,y)}$$

$$M_{VAT} = M_{Total} - M_{SAT}$$

Where in the corresponding equations:

- Rx, Ry is the resolution of DXA in X and in Y respectively
- $\rho_{Fat}$ is the value of the fat density

- [%G<sub>SAT</sub>] is average percentage of fat contained in the subdermal tissue
- $M_{Total}$ is the total fat mass determined by DXA

Once the values of subcutaneous fat (SAT) mass and visceral fat (VAT) mass are obtained, in the case of obtaining negative values of visceral fat (VAT) mass (which would imply an overestimation of subcutaneous fat (SAT) mass), the value of visceral fat (VAT) mass is corrected to zero.

The corrected total amount of visceral fat (VAT) mass is the sum of the corrected amount of visceral fat (VAT) mass per pixel underlying the abdominal cavity:

$$M'_{VAT} = \sum_{Cavity} M'_{VAT(x,y)}$$

Therefore, the corrected total amount of subcutaneous fat (SAT) mass is obtained in the following way:

$$M'_{SAT} = M_{Total} - M'_{VAT}$$

The estimation is carried out based on the 3D reconstruction, the geometric modelling of the abdominal cavity and the distribution of the fat content per pixel and a percent of the fat mass and the processed fat obtained from the corresponding signals of the DXA scan.

## DESCRIPTION OF THE DRAWINGS

As a complement to the description provided herein and for the purpose of helping to make the characteristics of the invention more readily understandable, in accordance with a preferred practical exemplary embodiment thereof, said description is accompanied by a set of drawings constituting an integral part of the same, which by way of illustration and not limitation represent the following:

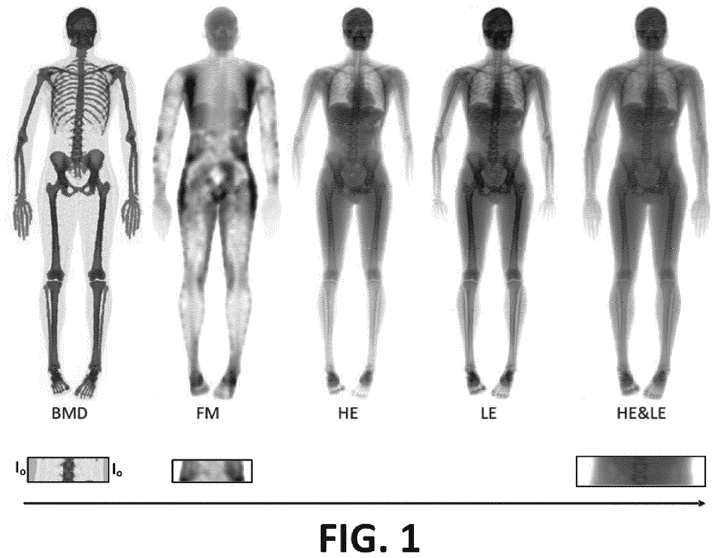

Figure 1 shows the main radiological images obtained by means of the DXA technique.
Figure 2 shows an illustration wherein the region of interest (ROI) is defined in a medical image.

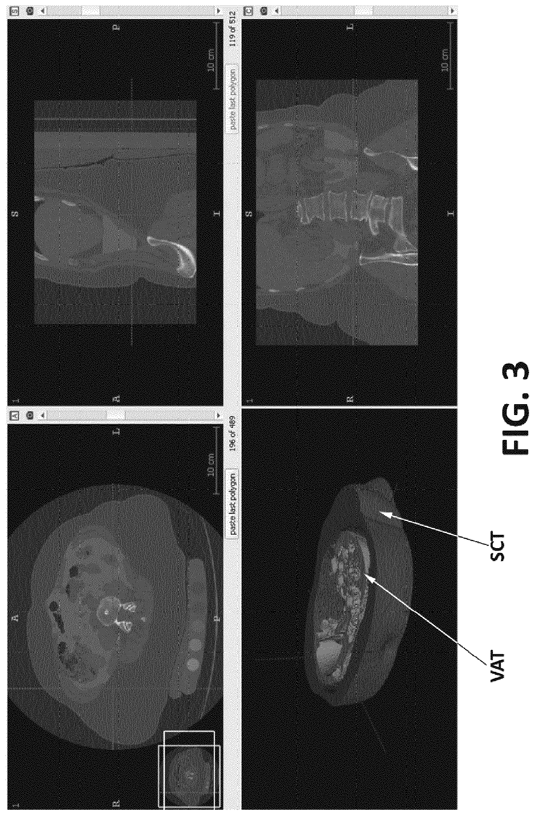

Figure 3 shows a series of illustrations wherein the segmentation of Computerized Tomography (CT) is shown.
Figure 4 shows the reconstruction of the depth of the patient's body, with which the abdominal depth will be estimated.
Figure 5 shows the correlation between the thickness of the tissue corresponding to subcutaneous fat (SAT), in the lower part of the ribs, and the depth of tissue corresponding to subcutaneous fat (SAT).
Figure 6 shows the depth profile of abdomen H and the depth profile of abdominal cavity h.
Figure 7 shows the profile of the difference between the functions H and h, the thickness of the tissue corresponding to subcutaneous fat (SAT).
Figure 8 shows the geometric model for reconstructing the patient's abdomen and abdominal cavity, pixel by pixel.

## PREFERRED EMBODIMENT OF THE INVENTION

In a preferred embodiment, the object of the invention is based on a series of DXA images extracting from the same characteristics inherent to the DXA type of measurement: pixel size (resolution), number of pixels in X and Y (transverse and longitudinal axis, respectively, the patient being in the supine position), speed, etc. that result in a DXA data matrix. For each pixel of the DXA data matrix, the data on the intensity of the high and low energy peaks is saved, the first of which expresses attenuation mainly through soft tissues, with different attenuation depending on the fat percentage, and the second which expresses attenuation mainly through bone tissues. This data is used along with the data on the air-attenuation intensity (in which there is no obstacle to the flow of radiation between the emitter and the detector). The different attenuation of the two energy peaks enables the main components of the body composition in a model of three body compartments to be recognized, according to the specific attenuation coefficient for each molecular type, namely: bone mass (mineral content of the bone structures of the skeleton and eventual calcified foci), fat mass (adipose tissue) and lean mass (group of non-bone and non-fat soft tissues that correspond to fluids, viscera and muscle mass). The group of pixels of a 2-dimensional matrix (X,Y) enables a distribution map of the composition of the body to be defined, whether it be human, an animal model or even biological parts that basically contain the three components described (Bone, Fat, Lean Mass). The object of the invention relates to a process wherein the assumption is made that the tissue composition (by radiological absorption) should be calculated in a region of interest that has its own geometry for each human subject, animal model or anatomical part to be analyzed. The object of the invention identifies the critical elements of reference (due to their special radiological behavior) for calculating the amount of fat mass, which are located in different body compartments. The application is intended to quantify the fat located inside the abdominal cavity (basically mesenteric fat, but called visceral fat or visceral adipose tissue, VAT), or even located in other parts of the human body, such as the infiltration of muscle groups.

To describe the process object of the invention, it is worth identifying the tasks of:

a) Recognition, elaboration and translation of the DXA radiological signals.
b) Identification and calculation of dimensions of the region of interest,
c) Identification of the subject-specific abdominal geometry model,
d) Recognition and calculation of subcutaneous fat and intracavitary fat.

To perform these calculations, images based on the different radiological absorption of the body components are reconstructed and wherein recognition algorithms of anatomical elements of reference are established. The sequence of signals extracted from the files generated by a system that uses DXA technology leads to special "radiological" images that are listed as follows:

I. Images of the high energy (HE) and low energy (LE) signals. A conventional combination, used in the state of the art, of these two signals, attained from the log ratio by dividing the high energy (HE) attenuation by the low energy (LE) attenuation, enables the different components to be identified by the different radiological absorption of each molecular component. The ratio calculated in this way is called the "R" index and is directly proportional to the content of fat present in each pixel.

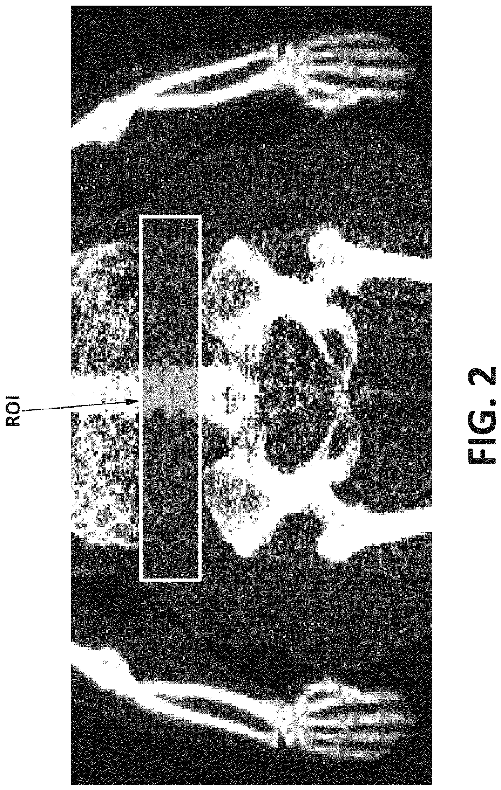

II. Bone mineral density (BMD) images obtained from the HE and LE signals that, due to the higher attenuation thereof derived from the presence of mineral images, identify the radiological absorption of the bone tissue located in a region of interest (hereinafter, ROI), much higher than the nearby soft tissues. This identification takes place by comparing the different behaviors in the energy peaks. This specific contrast between bones and soft tissues enables the selection in the ROI of the sector of the abdomen where there is a smaller ratio of bone structures (only the segment of the lumbar spine) and it is known in the state of the art as "android area". This ROI is delimited between the upper part of the iliac crests and the lower part of the ribs as shown in Figure 2.

III. Fat mass (FM) distribution image: This image provides a map of the fat mass. This is determined based on the HE and LE according to the methodologies established in the state of the art.

IV. Combined HE & LE image: At this point, an image is generated that is the combination of the two-corresponding high energy (HE) and low energy (LE) signals, with the aim of enhancing the contrast between subcutaneous tissue on the sides of the image and the abdominal cavity in the middle of the image, removing discontinuities and ultimately facilitating the search for geometries similar to the patients studied. Thus, a mathematical function is used with the HE and LE signal values to enhance the contrast thereof [for example, division between the HE logarithm and the LE logarithm (HE and LE are scalars of the X-ray signal)], which enables the contours to be clearly determined, in which the limits of the edge of the compartments to be quantified are defined. Subsequently, the method object of the invention processes the information of the contours, for example, by means of the smoothing of edges (anti-aliasing), so that there is no discontinuity in the limits detected, generating a file with smoothed contours without discontinuities therebetween and having a uniform contour. With said contours, now more clearly defined and without discontinuities, specific parameters of the patients are then calculated: abdominal cavity diameter at each height "y", external abdominal diameter at each height "y" and subcutaneous tissue thickness on the sides. Thus, the contrast between the subcutaneous tissue on the sides and the cavity in the middle is then enhanced. In this way, it is easier to detect the location of the external limit of the cavity, and this serves to calculate the diameter of the cavity and the thickness of the subcutaneous tissue with greater accuracy. As a result, the contrast is increased in order to determine the cavity.

V. Density processed fat (DPF) image: This image shows the distribution of the percentage of fat and it is determined by following methodologies commonly used in the state of the art.

The identification of the ROI in the case of the abdomen aims to define limits of reference in each subject that can always be reproduced when they have a smaller bone component ratio.

The dimensions and shape of the different areas in the body sector of interest are essential in order to locate the different body components.

Using the bone density (BMD) image, the region of interest (ROI) is selected, which comprises a group of pixels.

Each one of said pixels of the region of interest (ROI) can be located by means of coordinates (x,y) of the acquisition plane, the x-axis being in a transverse direction to the body and the y-axis being in a longitudinal direction.

The same region of interest (ROI) selected in the bone density (BMD) image is extended to the rest of the radiological images, also made up of a group of pixels, which can be located by the same coordinates (x,y), meaning that, hereinafter, reference is made to a pixel of coordinates (x,y) regardless of the type of image, whether it is a high energy (HE) image, a low energy (LE) image, a bone density (BMD) image, a fat mass (FM) distribution image, a combined HE&LE image, a processed fat (DPF) image or any other group of values associated with these coordinates derived or resulting from any type of mathematical treatment.

The dimensions of each pixel, in square millimeters, are given by the resolution (x,y) of the instrument on the acquisition plane of the DXA radiological signal, for example, it is 2.4 on the x-axis and 3.0375 mm on the y-axis for a DXA full-body image.

ROI depth-thickness: It corresponds to the calculation of anthropometric data on the depth or thickness in each pixel in order to define the abdominal geometry, identifying the shape and size model of the abdominal cavity that best fits the subject object of measurement. The thickness is automatically calculated, pixel by pixel, generating a depth value that is subsequently used to calculate the final reconstruction of the abdominal ROI or other regions of the patient.

The parameterization of the calculation of the thickness or depth of each pixel in the cells wherein only soft tissues are identified obviously enables the absolute calculation of the fat component and the non-fat components (lean tissues) by subtraction. By having the fat mass (FM), the density of processed fat (DPF) and using methodologies known in the state of the art, the amount of lean mass (LM) is obtained.

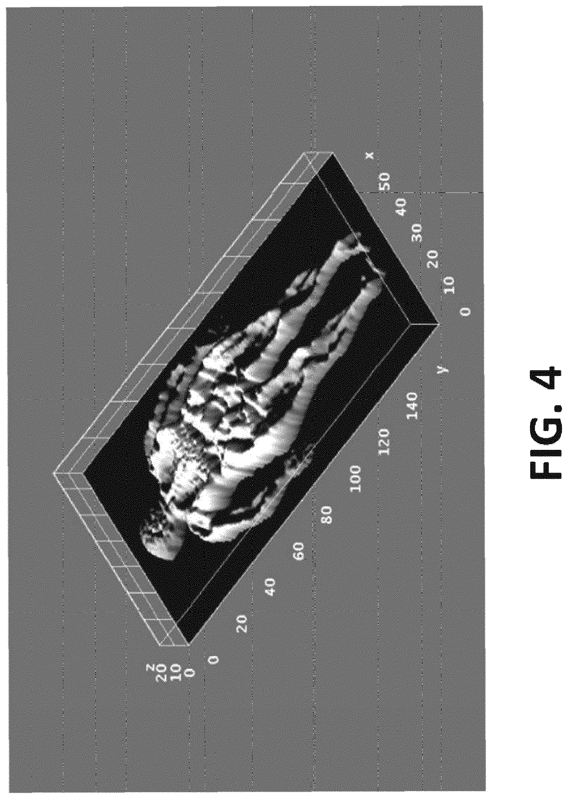

By knowing the pixel dimensions that are given by the resolution of the DXA image capturing instrument and by means of the volume ("density") coefficients of the fat component and of the lean component, the abdominal depth in each pixel (Figure 4) is estimated, selecting the maximum value as an estimation of the abdominal depth of the patient. Alternatively, this abdominal depth can be obtained by means of the physical anthropometric measurement of the patient.

In the abdominal cavity, there are also two other components, which are the bone mass and the air located inside the intestinal loops. These elements have an impact on the calculation of the average abdominal thickness, but their impact is corrected by the assumption of the minimum thickness of the cells and experimental correlations.

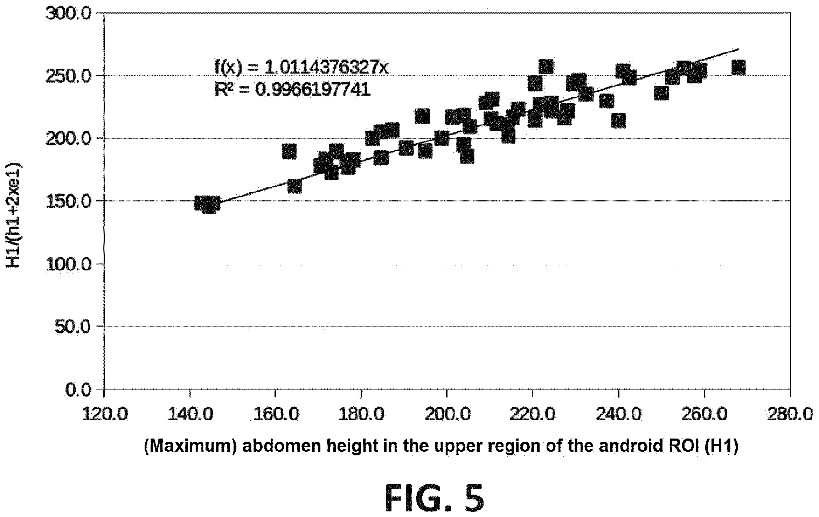

To obtain the depth of the abdominal cavity, a correlation based on the reading or measurement of the thickness of subcutaneous fat (SAT) in the upper part of the ROI, the lower part of the ribs, is used (Figure 5).

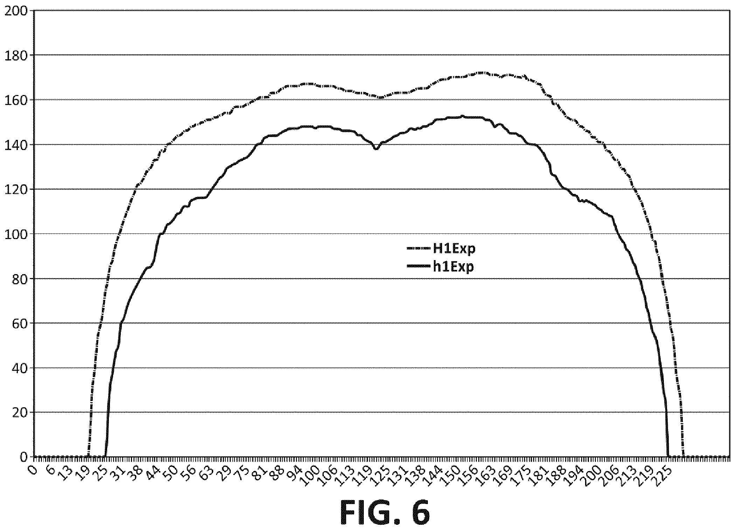

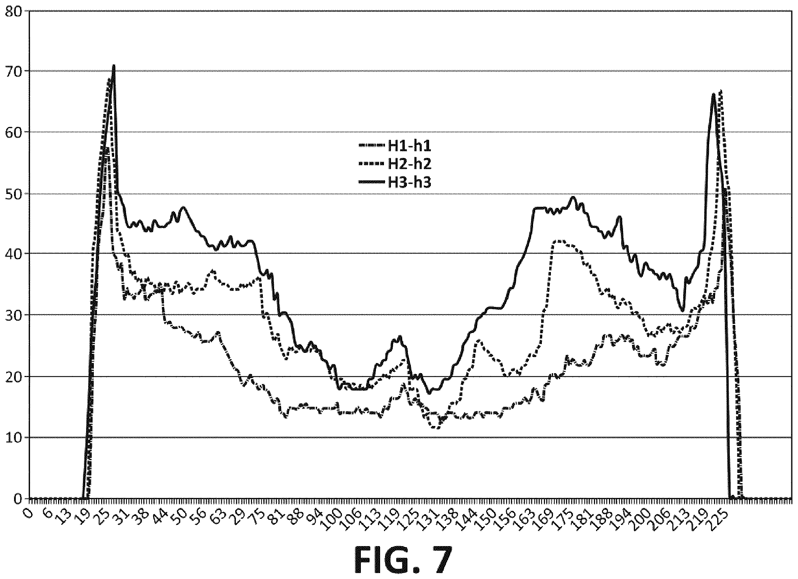

One pixel represents a projection on the plane (x,y) of all the fat and non-fat "material" found in the anteroposterior direction (z-axis). Therefore, by having the total fat percentage, it is only required to know the depth of the abdomen H at the height of that pixel in order to calculate the amount (in grams) of total fat. Likewise, if we know the (total) thickness of subcutaneous tissue, we can calculate the amount of subcutaneous fat mass and, by finding the difference, the amount of visceral fat mass. The (total) thickness of subcutaneous tissue in a given pixel is nothing more than the difference between "H" and the depth of the abdominal muscle cavity "h" (Figure 4 and Figure 6 and Figure 7).

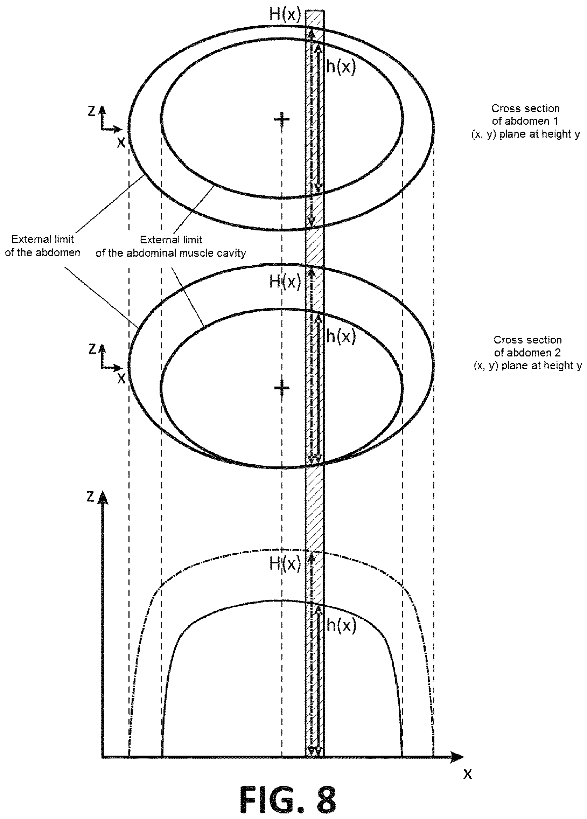

To carry out the method for quantifying intra-abdominal fat object of the invention, the recognition of the 3D volumetric model of the abdomen of the patient is carried out. To do so, the method of the invention is based on a search for previously identified geometric models that have the greatest possible similarity to the patient to be analyzed and that have been previously stored in a database with all of the characteristics thereof. Each specific geometry of each of the volumetric models can be described by means of the abdomen depth functions H(x,y) and external abdominal wall depth functions h(x,y) (Figure 8).

These functions were previously obtained from high resolution CTs of the abdominal area. The external abdomen and abdominal wall limits were obtained by means of the segmentation of the CTs, finding the depths H(x,y) and h(x,y). In the segmentation process, the tissue located immediately under the skin with radiological behavior equivalent to fat (values close to 150 Hounsfield units) is identified as an area of subcutaneous fat (SAT), while inside the abdominal cavity, the material with similar radiological behavior is identified as visceral fat (VAT).

The FM images provide the total (visceral and subcutaneous) fat mass in each pixel of the image of the abdomen. The pixel represents a projection on the plane (x, y) of all the fat and non-fat tissue found in the anteroposterior direction (z-axis). Therefore, by having the total fat percentage, we only need to know the depth of the abdomen H at the height of that pixel in order to calculate the amount (in grams) of total fat. Likewise, if we know the (total) thickness of subcutaneous tissue, we can calculate the amount of subcutaneous fat mass and, by finding the difference, the amount of visceral fat. The (total) thickness of subcutaneous tissue is nothing more than the difference between "H" and the depth of the abdominal muscle cavity "h".

The three-dimensional dimensions with the thickness profile of the pixels enable the most similar collected geometry of the CTs and that fits the best to be recognized (for example, ellipse, hyperellipse of the order n with n between 2 and 3, Spiric sections for the navel area, etc.).

With the data extracted from the DXA file on the fat content, definition of contours, internal abdominal cavity limits and abdominal thickness, the main calculation module is executed, where densitometry is first loaded in the bone mineral density (BMD) modality thereof, and after selecting the region of interest (ROI) comprising the abdominal area located between the lower part of the ribs and the upper part of the iliac bones, said patient is then analyzed with anatomical reference criteria, distinguishing the fat mass located in the internal cavity as visceral fat (VAT) and the peripheral fat in external limits as subcutaneous fat (SAT). In this way and due to the depth calculation of the 2D image coming from densitometry (DXA), a three-dimensional model of the abdomen of the patient is generated.

The component located between the skin and the abdominal muscle wall (behavior with a higher ratio of lean mass) on each side of the ROI is interpreted as subcutaneous fat given the excellent correlation thereof (Figure 5) with the fat identified in CTs and it is the basis for estimating the group of subcutaneous fat in the ROI. The visceral fat is obtained by means of subtraction from the total fat calculated in the ROI from the FM image.

The new 3D reconstruction model was attained with the abdominal thickness profile and with abdominal cavity dimensions calculated from the stored geometry data based on the CT atlas, with the two main parameters that are the abdominal cavity width and depth. Once the anthropomorphic geometry is reconstructed, and based on the dimensions calculated, the fat content is analyzed, interpreting the fat mass in the abdominal cavity as visceral fat (VAT), and the fat peripheral to the intra-abdominal ROI as subcutaneous fat (SAT), these values ultimately being the resulting values of subcutaneous and visceral fat of the study.

**Claims**

1. A method for quantifying visceral fat (VAT) mass of a living being, the method comprising taking a series of radiological images obtained by means of dual-energy X-ray absorptiometry (DXA), the method being **characterized in that** the radiological images comprise:

   • bone density (BMD) images, related to bone density,
   • fat mass (FM) images comprising information about an amount of fat mass in each pixel of the image,
   • a combined image generated from two signals, one high energy (HE) signal and one low energy (LE) signal, and
   • density processed fat (DPF) images

   and **in that** it comprises:

   • selecting a region of interest (ROI) based on a contrast between bones and soft tissues of the bone density (BMD) images,
   • enhancing the contrast in the region of interest (ROI) between the subcutaneous tissue and the abdominal cavity by combining high energy (HE) images and low energy (LE) images,
   • determining the external contours of the abdomen and of the abdominal cavity from the high energy (HE) images and low energy (LE) images by a combination thereof, generating a combined image with enhanced contrast,
   • estimating the maximum abdominal depth from the amount of fat mass and lean mass obtained from the density processed fat (DPF) images and the fat mass (FM) images and the densities thereof,
   • estimating the depth of subcutaneous fat tissue (SAT) by means of a correlation with the thickness of subcutaneous fat tissue (SAT) in a region of interest (ROI),
   • three-dimensionally reconstructing an abdominal depth H(x,y) and the abdominal cavity h(x,y) of the patient, wherein said reconstruction comprises:

      ◦ detecting contours by means of enhancing the contrast between the subcutaneous fat tissue (SAT) of the sides of the image resulting from the combination of the high energy (HE) images and low energy (LE) images and the abdominal cavity in the middle of the image and a smoothing of discontinuities,
      ◦ estimating a tissue depth value corresponding to subcutaneous fat (SAT) and a maximum abdominal depth value H(x,y),
      ◦ determining contour geometries and estimations of abdominal depth H(x,y) and of abdominal cavity depth h(x,y), from the geometries obtained by means of Computerized Tomography,

   • calculating an amount of subcutaneous fat (SAT) mass for each pixel of coordinates (x,y) of the region of interest (ROI) such as $M_{SAT(x,y)}$ according to:

$$M_{SAT\,(x,y)} = [\%G_{SAT}].Rx.Ry.\rho_{Fat}.\,(H(x,y) - h(x,y))$$

   where:

      ▪ $\rho_{Fat}$ is the value of the fat density,
      ▪ $[\%G_{SAT}]$ is a percentage value of subcutaneous fat (SAT) mass in the subdermal tissue,

- Rx, Ry is the pixel size of DXA in X and in Y respectively and the product thereof given the dimensions of the pixel (x,y), the density of fat $\rho_{Fat}$ and the ratio of fat in the subdermal tissue [%$G_{SAT}$], obtained by measuring the ratio of subcutaneous fat (SAT) mass on the sides of the ribs in the density processed fat (DPF) image,

• calculating an amount of visceral fat (VAT) mass for each pixel of coordinates (x,y) comprised in the region of interest (ROI) by subtracting the amount of subcutaneous fat (SAT) mass for each pixel of coordinates (x,y) comprised in the region of interest (ROI) from the total amount of fat mass obtained from the fat mass (FM) image,
• obtaining an image resulting from the distribution of subcutaneous fat (SAT) mass and an image resulting from the distribution of visceral fat (VAT) mass;
• adding up the amount of visceral fat (VAT) mass and subcutaneous fat (SAT) mass in each pixel of coordinates (x,y) of the image resulting from the distribution of subcutaneous fat (SAT) mass and of the image resulting from the distribution of visceral fat (VAT) mass, respectively, for the entire region of interest (ROI), in order to obtain a total value of the visceral fat (VAT) mass and a total value of the amount of subcutaneous fat (SAT) mass.
• correcting to 0 the amount of visceral fat (VAT) mass in those pixels of coordinates (x,y) whose difference between the total fat mass, in a given pixel and obtained in the fat mass (FM) image, and the subcutaneous fat (SAT) mass in said pixel gives a negative value of visceral fat (VAT) mass,
• adding up the corrected amount of visceral fat (VAT) mass in the entire region of interest (ROI), and
• obtaining the total corrected amount of subcutaneous fat (SAT) mass by subtracting the total corrected amount of visceral fat (VAT) mass from the total amount of fat mass obtained from the fat mass (FM) image.

2. The method according to claim 1, **characterized in that** the region of interest (ROI) is delimited between the upper part of the hips and the lower part of the ribs.

3. The method according to claim 1, **characterized in that** it comprises reconstructing the region of interest (ROI) from a 2D image of dual-energy X-ray absorptiometry (DXA) extracted by means of densitometry and from geometries obtained from a 3D image extracted by means of Computerized Tomography.

4. The method according to claim 3, **characterized in that** it further comprises defining a resolution of the 2D image of dual-energy X-ray absorptiometry (DXA).

5. The method according to any one of claims 1 to 4, **characterized in that** it further comprises defining an anthropometric depth data for each pixel of the DXA image, this anthropometric depth data being the result of measuring an abdominal depth of the subject.

6. The method according to claim 5, **characterized in that** the anthropometric depth data is measured while the subject is in the supine position.

7. The method according to any one of the preceding claims, **characterized in that** the contours are determined by the image resulting from the combination of the high energy (HE) images and low energy (LE) images, enhancing the contrast between the subcutaneous tissue of the sides of the image and the abdominal cavity in the middle of the image and smoothing the limits detected to eliminate discontinuities, thus making it easier to detect the contours that define limits of the edge of the compartments to be quantified.

8. The method according to any one of the preceding claims, **characterized in that** it further comprises generating a graphical distribution of the amount of visceral fat (VAT) mass and amount of subcutaneous fat (SAT) mass based on the amounts estimated in each pixel of the image corresponding to the abdomen.

**Patentansprüche**

1. Verfahren zum Quantifizieren der viszeralen Fettmasse (VAT) eines Lebewesens, wobei das Verfahren das Aufnehmen einer Reihe von radiologischen Bildern umfasst, die mittels Dual-Energy-Röntgenabsorptiometrie (DXA) erhalten werden, wobei das Verfahren **dadurch gekennzeichnet ist, dass** die radiologischen Bilder umfassen:

• Bilder der Knochendichte (BMD), die sich auf die Knochendichte beziehen,
• Bilder der Fettmasse (FM), die Informationen über eine Menge an Fettmasse in jedem Pixel des Bildes umfassen,

• ein kombiniertes Bild, das aus zwei Signalen erzeugt wird, einem Hochenergiesignal (HE) und einem Niedrigenergiesignal (LE), und
• Bilder des dichteverarbeiteten Fettes (DPF)

und dadurch, dass es umfasst:

• Auswählen einer Region von Interesse (ROI) basierend auf einem Kontrast zwischen Knochen und Weichgeweben der Bilder der Knochendichte (BMD),
• Verbessern des Kontrasts in der Region von Interesse (ROI) zwischen dem subkutanen Gewebe und der Bauchhöhle durch Kombinieren von Bildern mit hoher Energie (HE) und Bildern mit niedriger Energie (LE),
• Bestimmen der Außenkonturen des Abdomens und der Bauchhöhle aus den Bildern mit hoher Energie (HE) und den Bildern mit niedriger Energie (LE) durch eine Kombination davon, wodurch ein kombiniertes Bild mit verbessertem Kontrast erzeugt wird,
• Schätzen der maximalen Abdominaltiefe aus der Menge an Fettmasse und Magermasse, die aus den Bildern des dichteverarbeiteten Fettes (DPF) und den Bildern von Fettmasse (FM) und deren Dichten erhalten wird,
• Schätzen der Tiefe des subkutanen Fettgewebes (SAT) mittels einer Korrelation mit der Dicke des subkutanen Fettgewebes (SAT) in einer Region von Interesse (ROI),
• dreidimensionales Rekonstruieren einer Abdominaltiefe H(x,y) und der Bauchhöhle h(x,y) des Patienten, wobei die Rekonstruktion umfasst:

◦ Erkennen von Konturen durch Erhöhen des Kontrasts zwischen dem subkutanen Fettgewebe (SAT) der Seiten des Bildes, das sich aus der Kombination der Bilder mit hoher Energie (HE) und der Bildern mit niedriger Energie (LE) und der Bauchhöhle in der Mitte des Bildes und einer Glättung von Diskontinuitäten ergibt,
◦ Schätzen eines Gewebetiefenwerts, der dem subkutanen Fett (SAT) entspricht, und eines maximalen Abdominaltiefenwerts H(x,y),
◦ Bestimmen von Konturgeometrien und Schätzungen der Abdominaltiefe H(x,y) und der Bauchhöhlentiefe h(x,y) aus den mittels Computertomographie erhaltenen Geometrien,

• Berechnen einer Menge an subkutaner Fettmasse (SAT) für jedes Koordinatenpixel (x,y) der Region von Interesse (ROI) wie $M_{SAT}(x,y)$ gemäß:

$$M_{SAT}(x,y) = [\%G_{SAT}].Rx.Ry.\rho_{FAT}. (H(x,y) - h(x,y))$$

wobei:

- $\rho_{Fat}$ der Wert der Fettdichte ist,
- [%Gsat] ein prozentualer Wert der subkutanen Fettmasse (SAT) im subdermalen Gewebe ist,
- Rx, Ry die Pixelgröße von DXA in X bzw. Yund das Produkt davon unter Berücksichtigung der Abmessungen des Pixels (x,y), der Fettdichte $\rho_{Fat}$ und des Fettverhältnisses im subdermalen Gewebe [%Gsat] ist,

das durch Messen des Verhältnisses der subkutanen Fettmasse (SAT) an den Seiten der Rippen im Bild des dichteverarbeiteten Fettes (DPF) erhalten wird,
• Berechnen einer Menge an viszeraler Fettmasse (VAT) für jedes Pixel von Koordinaten (x,y), die in der Region von Interesse (ROI) enthalten sind, durch Subtrahieren der Menge an subkutaner Fettmasse (SAT) für jedes Pixel von Koordinaten (x,y), die in der Region von Interesse (ROI) enthalten sind, von der Gesamtmenge an Fettmasse, die aus dem Bild der Fettmasse (FM) erhalten wird,
• Erhalten eines Bildes, das sich aus der Verteilung der subkutanen Fettmasse (SAT) ergibt, und eines Bildes, das sich aus der Verteilung der viszeralen Fettmasse (VAT) ergibt;
• Addieren der Menge an viszeraler Fettmasse (VAT) und subkutaner Fettmasse (SAT) in jedem Pixel von Koordinaten (x,y) des Bildes, das sich aus der Verteilung der subkutanen Fettmasse (SAT) ergibt, sowie des Bildes, das sich aus der Verteilung der viszeralen Fettmasse (VAT) für die gesamte Region von Interesse (ROI) ergibt, um einen Gesamtwert der viszeralen Fettmasse (VAT) und einen Gesamtwert der Menge der subkutanen Fettmasse (SAT) zu erhalten.
• Korrigieren auf 0 der Menge an viszeraler Fettmasse (VAT) in denjenigen Pixeln von Koordinaten (x,y), deren Differenz zwischen der Gesamtfettmasse in einem gegebenen Pixel und erhalten in dem Bild der Fettmasse (FM), und der subkutanen Fettmasse (SAT) in dem Pixel einen negativen Wert der viszeralen Fettmasse (VAT) ergibt,

• Addieren der korrigierten Menge an viszeraler Fettmasse (VAT) in der gesamten Region von Interesse (ROI) und

• Erhalten der gesamten korrigierten Menge an subkutaner Fettmasse (SAT) durch Subtrahieren der gesamten korrigierten Menge an viszeraler Fettmasse (VAT) von der Gesamtmenge an Fettmasse, die aus dem Bild der Fettmasse (FM) erhalten wird.

2. Verfahren nach Anspruch 1, **dadurch gekennzeichnet, dass** die Region von Interesse (ROI) zwischen dem oberen Teil der Hüften und dem unteren Teil der Rippen abgegrenzt ist.

3. Verfahren nach Anspruch 1, **dadurch gekennzeichnet, dass** es das Rekonstruieren der Region von Interesse (ROI) aus einem 2D-Bild der Dual-Energy-Röntgenabsorptiometrie (DXA), die mittels Densitometrie extrahiert wird, und aus Geometrien umfasst, die aus einem mittels Computertomographie extrahierten 3D-Bild erhalten werden.

4. Verfahren nach Anspruch 3, **dadurch gekennzeichnet, dass** es ferner das Definieren einer Auflösung des 2D-Bildes der Dual-Energy-Röntgenabsorptiometrie (DXA) umfasst.

5. Verfahren nach einem der Ansprüche 1 bis 4, **dadurch gekennzeichnet, dass** es ferner das Definieren anthropometrischer Tiefendaten für jedes Pixel des DXA-Bildes umfasst, wobei diese anthropometrischen Tiefendaten das Ergebnis der Messung einer Abdominaltiefe des Subjekts sind.

6. Verfahren nach Anspruch 5, **dadurch gekennzeichnet, dass** die anthropometrischen Tiefendaten gemessen werden, während sich das Subjekt in Rückenlage befindet.

7. Verfahren nach einem der vorhergehenden Ansprüche, **dadurch gekennzeichnet, dass** die Konturen von dem Bild bestimmt werden, das sich aus der Kombination der Bilder mit hoher Energie (HE) und der Bilder mit niedriger Energie (LE) ergibt, wodurch der Kontrast zwischen dem subkutanen Gewebe der Seiten des Bildes und der Bauchhöhle in der Mitte des Bildes und Glätten der erkannten Grenzen, um Diskontinuitäten zu beseitigen, verstärkt wird, wodurch es einfacher wird, die Konturen zu erkennen, die Grenzen des Randes der zu quantifizierenden Kompartimente definieren.

8. Verfahren nach einem der vorhergehenden Ansprüche, **dadurch gekennzeichnet, dass** es ferner das Erzeugen einer grafischen Verteilung der Menge an viszeraler Fettmasse (VAT) und der Menge an subkutaner Fettmasse (SAT) auf der Grundlage der Mengen umfasst, die in jedem Pixel des zum Abdomen gehörenden Bildes geschätzt werden.

**Revendications**

1. Procédé pour la quantification de la masse de graisse viscérale (VAT) d'un être vivant, le procédé comprenant la prise d'une série d'images radiologiques obtenues au moyen d'absorptiométrie à rayons X en double énergie (DXA), le procédé étant **caractérisé en ce que** les images radiologiques comprennent :

• des images de densité osseuse (BMD), relatives à la densité osseuse,
• des images de masse grasse (FM) comprenant des informations sur une quantité de masse grasse dans chaque pixel de l'image,
• une image combinée générée à partir de deux signaux, un signal à haute énergie (HE) et un signal à basse énergie (LE), et
• des images de densité de graisse traitée (DPF)

et **en ce qu'**il comprend :

• la sélection d'une région d'intérêt (ROI) sur la base d'un contraste entre les os et les tissus mous des images de densité osseuse (BMD),
• l'amélioration du contraste dans la région d'intérêt (ROI) entre le tissu sous-cutané et la cavité abdominale en combinant des images à haute énergie (HE) et des images à basse énergie (LE),
• la détermination des contours externes de l'abdomen et de la cavité abdominale à partir des images à haute énergie (HE) et des images à basse énergie (LE) par une combinaison de celles-ci, générant une image combinée avec un contraste amélioré,

• l'estimation de la profondeur abdominale maximale à partir de la quantité de masse grasse et de masse maigre obtenue à partir des images de densité de graisse traitée (DPF) et des images de masse grasse (FM) et des densités de celles-ci,

• l'estimation de la profondeur de tissu de graisse sous-cutanée (SAT) au moyen d'une corrélation avec l'épaisseur de tissu de graisse sous-cutanée (SAT) dans une région d'intérêt (ROI),

• la reconstruction en trois dimensions d'une profondeur abdominale H(x,y) et de la cavité abdominale h(x,y) du patient, dans lequel ladite reconstruction comprend :

  ◦ la détection de contours au moyen de l'amélioration du contraste entre le tissu de graisse sous-cutanée (SAT) des côtés de l'image résultant de la combinaison des images à haute énergie (HE) et des images à basse énergie (LE) et la cavité abdominale au milieu de l'image et un lissage de discontinuités,
  ◦ l'estimation d'une valeur de profondeur de tissu correspondant à la graisse sous-cutanée (SAT) et d'une valeur de profondeur abdominale maximale H(x,y),
  ◦ la détermination de géométries de contour et d'estimations de profondeur abdominale H(x,y) et de profondeur de cavité abdominale h(x,y), à partir des géométries obtenues au moyen de tomographie informatisée,

• le calcul d'une quantité de masse de graisse sous-cutanée (SAT) pour chaque pixel de coordonnées (x,y) de la région d'intérêt (ROI) telle que $M_{SAT\,(x,y)}$ selon :

$$M_{SAT(x,y)} = [\%G_{SAT}].Rx.Ry.\rho_{GRAISSE}. (H(x,y) - h(x,y))$$

où :

  ▪ $\rho_{Graisse}$ est la valeur de la densité de graisse,
  ▪ [%Gsat] est une valeur en pourcentage de masse de graisse sous-cutanée (SAT) dans le tissu sous la peau,
  ▪ Rx, Ry est la taille de pixel de DXA dans X et dans Y respectivement et le produit de celui-ci étant donné les dimensions du pixel (x,y), la densité de graisse $\rho_{Graisse}$ et le rapport de graisse dans le tissu sous la peau [%Gsat], obtenus en mesurant le rapport de masse de graisse sous-cutanée (SAT) sur les côtés des côtes dans l'image de densité de graisse traitée (DPF),

• le calcul d'une quantité de masse de graisse viscérale (VAT) pour chaque pixel de coordonnées (x,y) compris dans la région d'intérêt (ROI) en soustrayant la quantité de masse de graisse sous-cutanée (SAT) pour chaque pixel de coordonnées (x,y) compris dans la région d'intérêt (ROI) à partir de la quantité totale de masse grasse obtenue à partir de l'image de masse grasse (FM),

• l'obtention d'une image résultant de la répartition de masse de graisse sous-cutanée (SAT) et d'une image résultant de la répartition de la masse de graisse viscérale (VAT) ;

• l'ajout de la quantité de masse de graisse viscérale (VAT) et de graisse sous-cutanée (SAT) dans chaque pixel de coordonnées (x,y) de l'image résultant de la répartition de masse de graisse sous-cutanée (SAT) et de l'image résultant de la répartition de masse de graisse viscérale (VAT), respectivement, pour toute la région d'intérêt (ROI), afin d'obtenir une valeur totale de la masse de graisse viscérale (VAT) et une valeur totale de la quantité de masse de graisse sous-cutanée (SAT).

• la correction à 0 de la quantité de masse de graisse viscérale (VAT) dans ces pixels de coordonnées (x,y) dont la différence entre la masse de graisse totale, dans un pixel donné et obtenue dans l'image de masse grasse (FM), et la masse de graisse sous-cutanée (SAT) dans ledit pixel donne une valeur négative de masse de graisse viscérale (VAT),

• l'ajout de la quantité corrigée de masse de graisse viscérale (VAT) dans toute la région d'intérêt (ROI), et

• l'obtention de la quantité totale corrigée de masse de graisse sous-cutanée (SAT) en soustrayant la quantité totale corrigée de masse de graisse viscérale (VAT) de la quantité totale de masse grasse obtenue à partir de l'image de masse grasse (FM).

2. Procédé selon la revendication 1, **caractérisé en ce que** la région d'intérêt (ROI) est délimitée entre la partie supérieure des hanches et la partie inférieure des côtes.

**3.** Procédé selon la revendication 1, **caractérisé en ce qu'**il comprend la reconstruction de la région d'intérêt (ROI) à partir d'une image 2D d'absorptiométrie à rayons X en double énergie (DXA) extraite au moyen de densitométrie et à partir de géométries obtenues à partir d'une image 3D extraite au moyen de tomographie informatisée.

**4.** Procédé selon la revendication 3, **caractérisé en ce qu'**il comprend en outre la définition d'une résolution de l'image 2D d'absorptiométrie à rayons X en double énergie (DXA).

**5.** Procédé selon l'une quelconque des revendications 1 à 4, **caractérisé en ce qu'**il comprend en outre la définition d'une donnée anthropométrique de profondeur pour chaque pixel de l'image DXA, cette donnée anthropométrique de profondeur étant le résultat de la mesure d'une profondeur abdominale du sujet.

**6.** Procédé selon la revendication 5, **caractérisé en ce que** la donnée anthropométrique de profondeur est mesurée tandis que le sujet est en position couchée.

**7.** Procédé selon l'une quelconque des revendications précédentes, **caractérisé en ce que** les contours sont déterminés par l'image résultant de la combinaison des images à haute énergie (HE) et des images à basse énergie (LE), améliorant le contraste entre le tissu sous-cutané des côtés de l'image et la cavité abdominale au milieu de l'image et lissant les limites détectées pour éliminer les discontinuités, facilitant ainsi la détection des contours qui définissent des limites du bord des compartiments à quantifier.

**8.** Procédé selon l'une quelconque des revendications précédentes, **caractérisé en ce qu'**il comprend en outre la génération d'une répartition graphique de la quantité de masse de graisse viscérale (VAT) et de masse de graisse sous-cutanée (SAT) sur la base des quantités estimées dans chaque pixel de l'image correspondant à l'abdomen.

**FIG. 1**

**FIG. 2**

**FIG. 3**

FIG. 4

EP 3 501 399 B1

**FIG. 5**

**FIG. 6**

**FIG. 7**

H(x)

h(x)

Z
X

Cross section
of abdomen 1
(x, y) plane at height y

External limit
of the abdomen

External limit
of the abdominal muscle cavity

H(x)

h(x)

Z
X

Cross section
of abdomen 2
(x, y) plane at height y

Z

H(x)

h(x)

X

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7725153 B2

- US 8483458 B2

**Non-patent literature cited in the description**

- **ALBANESE et al.** Clinical applications of body composition measurements using DXA. *Journal of Clinical Densitometry,* 2003
- **A MOHAMMAD et al.** Validity of visceral adiposity estimates from DXA against MRI in Kuwaiti men and women. *Nutrition & Diabetes,* 09 January 2017, vol. 7, e238

- Dual-Energy X-Ray Absorptiometry for Quantification of Visceral Fat. **SANJIV KAUL et al.** Obesity. Silver Spring, June 2012, vol. 20, 1313-8